(19) Europäisches Patentamt
European Patent Office
Office européen des brevets

(11) Publication number: **0 144 519**
**B1**

(12) # EUROPEAN PATENT SPECIFICATION

(45) Date of publication of patent specification: **10.06.87**

(51) Int. Cl.4: **C 07 C 149/247,** C 07 C 153/11

(21) Application number: **84109446.9**

(22) Date of filing: **08.08.84**

(54) **2',4'-Difluoro-4-hydroxy-(1,1'-diphenyl)-3-carboxylic derivatives of N-acetylcysteine and of S-carboxymethylcysteine having anti-inflammatory and mucolytic activity, process for their preparation and related pharmaceutical compositions.**

(30) Priority: **09.08.83 IT 2248683**

(43) Date of publication of application:
**19.06.85 Bulletin 85/25**

(45) Publication of the grant of the patent:
**10.06.87 Bulletin 87/24**

(84) Designated Contracting States:
**AT DE GB IT NL SE**

(56) References cited:
**EP-A-0 080 229**
**FR-A-2 068 398**
**US-A-3 965 167**

(73) Proprietor: **Laboratori Guidotti S.p.A.**
**Via Trieste 40**
**I-50100 Pisa (IT)**

(72) Inventor: **Cerbai, Guido**
**Via Brodolini 10**
**I-56100 Pisa (IT)**

(74) Representative: **Struif, Bernward, Dipl.-Chem. Dr. et al**
**Patentanwaltsbüro Tiedtke, Bühling, Kinne Grupe, Pellmann, Grams, Struif Bavariaring 4**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 144 519 B1

**Description**

The present invention relates to novel compounds having general formula

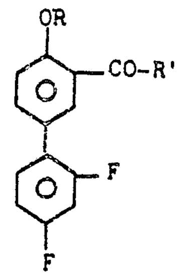

1

wherein R and R′ have the following meanings:

R = H, $-COCH_3$, $-COOC_2H_5$, $-COOCH_2-CCl_3$

R′ = $-S-CH_2-CH(NHCOCH_3)-COOH$; $-NH-CH(CH_2-S-CH_2-COOH)-COOH$

More specifically, the present invention relates to S-[2′,4′-difluoro-4-hydroxy-(1,1′-diphenyl)-carbonyl]-N-acetyl-L-cysteine and N-[2′,4′-difluoro-4-hydroxy-(1,1′-diphenyl)-carbonyl]-carboxymethylcysteine and their O-carbonyloxyalky and O-acetyl derivatives, as well as their non toxic and pharmaceutically acceptable salts with organic and inorganic bases. The present invention also relates to their preparation method and to their therapeutical use in the treatment of the catarrhal diseases of the tracheobronchial tract, by administration either orally and/or by rectal, aerosol and injective route.

The pathology of the respiratory system is among the very first in the classification of the main causes of illness.

Among the respiratory diseases, the most frequent are undoubtedly those affecting the tracheo-bronchial tract, both in the acute and in the chronic form.

Leaving the pure broncho-spastic forms out of consideration as being percentually a minority, these diseases are characterized by a phlogystic state of the tracheobronchial mucosa and by a frequently exceedingly viscous secretion. The pharmacological therapy of tracheo-bronchitis exempt from complications does classically contemplate the use, from one side of mucolytic fluidifying the balsamic agents and, from the other side, of anti-phlogistic, and anti-pyretic agents endowed with analgesic properties taking also into account that the acute influenzal and parainfluenzal form as commonly found often are accompanied by fever and arthromyalgia.

From the above considerations the utility appears of getting a drug capable of simultaneously acting on the phlogistic and catarrhal component, also providing an anti-pyretic and analgesic activity.

The compounds of the present invention in fact combine mucolytic properties with an anti-inflammatory, analgesic and anti-pyretic activity; they are represented by the formula 1 and are thus thioesters and amides comprising two molecular moyeties, that of the 2′-4′-difluoro-4-hydroxy-(1,1′-diphenyl)-3-carboxylic acid, (also known under the D.C.I. diflunisal), which is a substance known for its anti-inflammatory properties, and that of N-acetylcysteine or of S-carboxymethylcysteine, which are substances known for their mucolytic properties.

As already mentioned the present invention does also contemplate the non toxic and pharmaceutically acceptable salts of the compounds of the present invention: among them of particular interest is the lysine salt of compound (5).

The compounds of formula 1, wherein R′ is $-S-CH_2-CH(NHCOCH_3)-COOH$ are prepared by converting the 2,4′-difluoro-4-hydroxy-(1,1′-diphenyl)-3-carboxylic acid into the corresponding mixed anhydrides by the reaction with 2,2,2-trichloroethylchlorocarbonate or ethylchlorocarbonate in the presence of an acid acceptor, and condensation of these intermediates with N-acetylcysteine, the O′alkylcarbonate thioesters being then hydrolysed to the corresponding phenolic thioesters, as shown in the scheme I.

An advantageous modification of the method for the preparation of the final compound *5* consists in preparaing the mixed anhydride *3* (with $R''=CH_2-CCl_3$) with a like process, in the presence of an excess both of trichloroethylchlorocarbonate and of triethylamine, so that the mixed anhydride *3* is isolated in a sufficiently pure form, the latter being then reacted with N-acetyl-L-cysteine, still in presence of triethylamine. By this route, through a subsequent addition of triethylamine excess, the product *5* is isolated as the triethylamine salt (5′), whereby the hydrogenolysis step of the compound of formula 4 to the final product *5* is eliminated.

Both processes are shown in the scheme I.

The compounds of formula 1 wherein R' is —NH—CH—COOH

$$\begin{array}{l} -NH-\underset{\displaystyle |}{CH}-COOH \\ \quad\quad\;\; CH_2-S-CH_2COOH \end{array}$$

are prepared through the condensation of the O-acetyl derivative of 2',4'-difluoro-4-hydroxy-(1,1'-diphenyl)-3-carboxylic acid with the dimethyl ester of S-carboxymethylcysteine in the presence of dicyclohexylcarbodiimide and alkaline hydrolysis of the thus obtained amide derivative as shown in the scheme II. In this scheme the preparation is also shown of the compound 1 wherein

$$R = -COCH_3 \text{ and } R' = -S-CH_2-\underset{\displaystyle NHCOCH_3}{\underset{|}{CH}}-COOH$$

through the condensation of the acid chloride of 2',4'-difluoro-4-hydroxy-(1,1'-diphenyl)-3-carboxylic acid with N-acetylcysteine.

SCHEME I

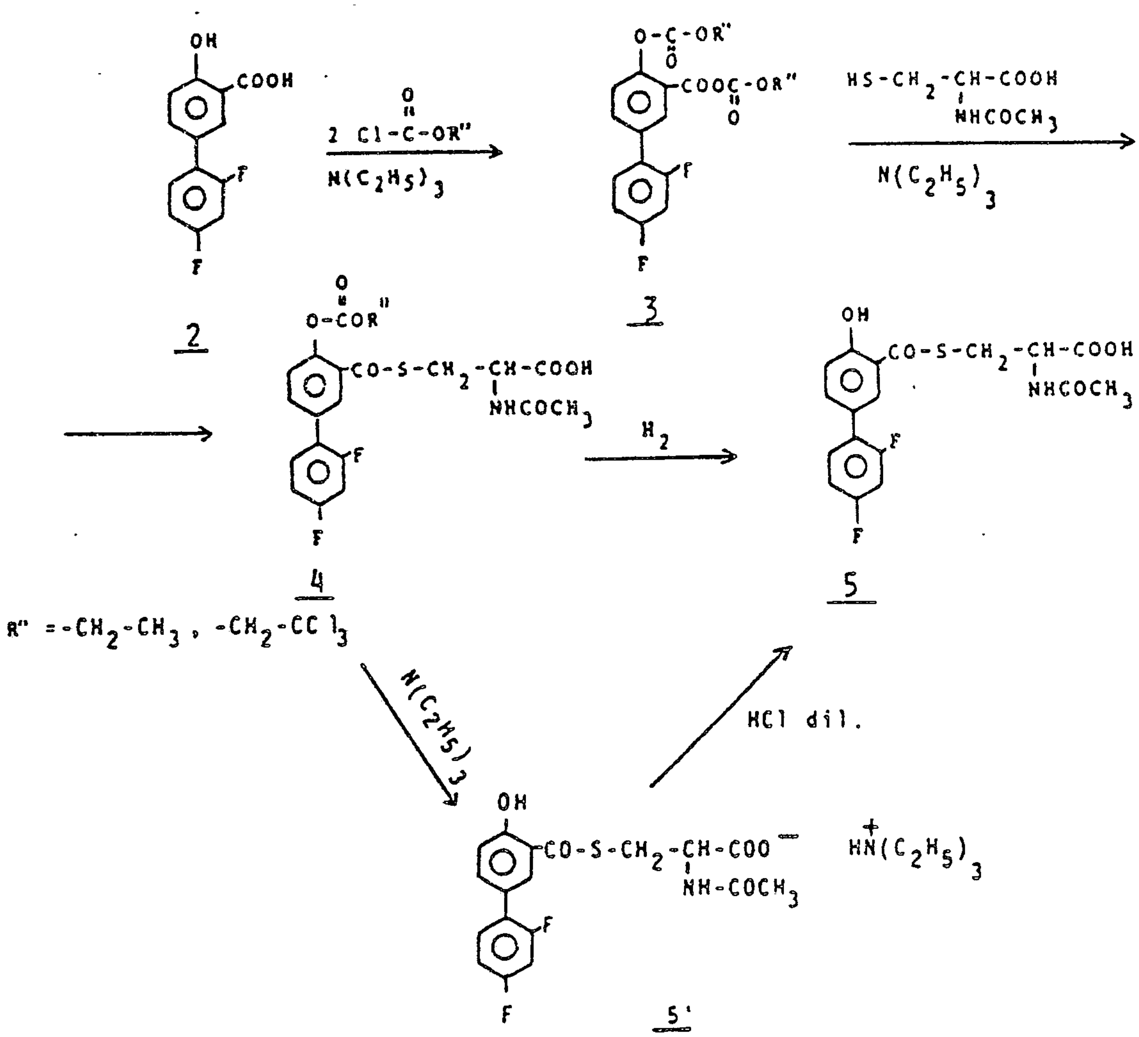


2 3 4 5 5'

R'' = -CH2-CH3, -CH2-CCl3

SCHEME II

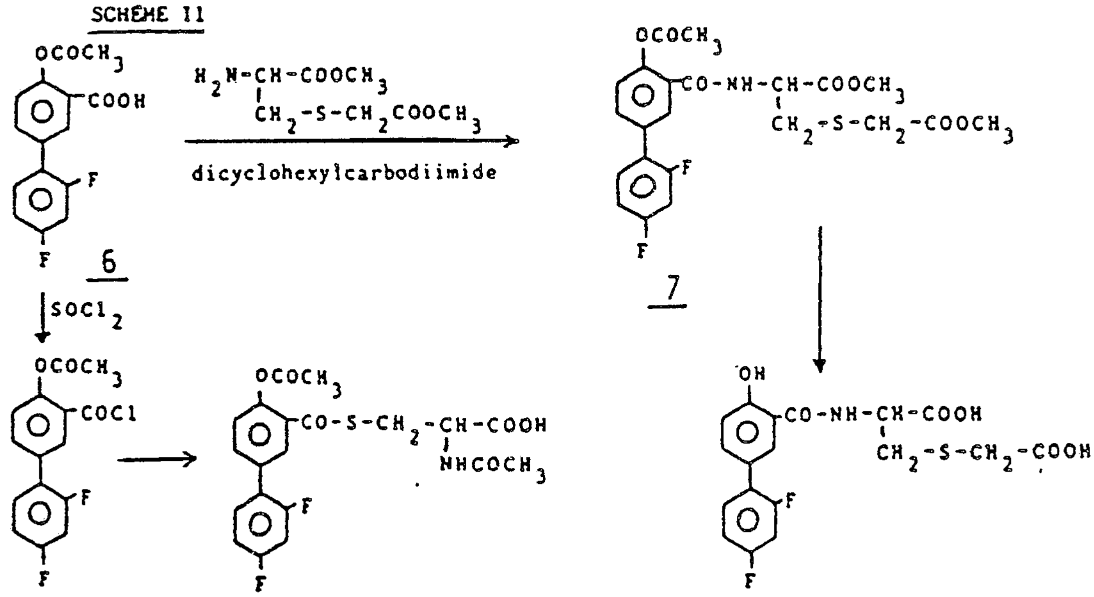


6 7

The pharmaco-toxicological properties of the compounds 1 have been studied in comparison with diflunisal and with N-acetylcysteine, in order to evaluate the pharmacological and toxicological properties of the compounds of the invention with respect to those of the starting compounds. The results relating to

the compound *5* (see scheme I) are hereinafter reported, only for illustrative and not for limiting purpose. The pharmacological evaluation in the conventional test of anti-inflammatory, analgesic, anti-pyretic activity (carrageen induced oedema in the rat, phenylquinone induced writhing in the mouse, baker yeast induced hyperpyrexy in the rat) demonstrated that the compound 5, at equimolar doses, has activity like that of diflunisal (as shown in the table 1), whereas in the tests of mucosecretodynamic activity, (fluorescein secretion in the respiratory tract of the mouse and mucus production in the rabbit), the S-[2',4'-difluoro-4-hydroxy-(1,1'-diphenyl)-3-carbonyl]-N-acetyl-L-cysteine (compound 5) has an activity comparable with that of N-acetylcysteine.

TABLE 1

Anti-inflammatory and analgesic activity of the compound 5, namely S-[2',4'-difluoro-4-hydroxy-(1,1'-diphenyl)-3-carbonyl]-N-acetyl-L-cysteine, in comparison with diflunisal in the carrageen induced paw oedema in the rat (a) and in the phenylquinone induced writhing in the mouse (b). (10 animals).

| TREATMENT | DOSE mmol $kg^{-1}$ p.o. | INIBITION % (medium value ± s.e.) anti-inflammatory activity (a) | analgesic activity (b) |
|---|---|---|---|
| Compound 5 | 0.040 | 21.8 ± 6.4 | 25.5 ± 9.2 |
| | 0.120 | 34.5 ± 5.5 | 29.3 ± 14.0 |
| | 0.400 | 42.4 ± 7.5 | 35.5 ± 12.0 |
| Diflunisal | 0.040 | 29.6 ± 5.4 | 9.5 ± 6.2 |
| | 0.120 | 50.3 ± 2.0 | 16.6 ± 15.9 |
| | 0.400 | 53.9 ± 4.7 | 39.6 ± 9.8 |

(a) Compounds administered 1h before the carrageen; oedema inhibition evaluated 3h after the carrageen administration.
(b) Products administered 2h before phenylquinone.

From the toxicologycal point of view the LD50 of compound 5 has been evaluated in comparison with diflunisal, with N-acetylcysteine and with an equimolar mixture of diflunisal and N-acetyl cysteine.

As shown in table 2, the compound 5 is in molar terms about 3 times less toxic than diflunisal. It is interesting to note that compound 5 has a definitely lower toxicity than the equimolar mixture of the two single components.

TABLE 2

Acute toxicity of S-[2',4'-difluoro-4-hydroxy-(1,1'-diphenyl)-3-carbonyl]-N-acetyl-L-cysteine (compound 5) by oral route in the female mouse in comparison with diflunisal, N-acetylcysteien and an equimolar mixture of diflunisal and N-acetylcysteine.
LD50 and related 95% reliability limits.

| Compound 5 | Diflunisal | N-acetylcysteine | Diflunisal+ N-acetyl-cisteine (equimolar a mounts) |
|---|---|---|---|
| $LD_{50}$mmol $kg^{-1}$ 9.30 (8.61—10.04) | 3.59 (3.15—4.09) | 32.4 (24.1—43.6) | 3.94 (2.97—5.22) |
| mg $kg^{-1}$ 3677 (3404—3970) | 898 (788—1023) | 5288 (3931—7112) | 986 (743—1306) diflunisal<br>643 (485—852) N-acetylcisteine |

Such a surprising property can be probably explained in view of the pharmacokinetic properties of the compound, as described hereinafter.

Other toxicological features of the compound 5 have been also studied, more particularly the histolesivity (intramuscular injection in the sacrospinalis muscle of rabbit) and the gastolesivity (ulcers after oral administration in the rat).

In both cases compound 5 revealed a scarce or no lesivity, anyhow definitely lower, at equimolar doses, than the high one of diflunisal.

Lastly, some pharmacokinetic properties of the compound 5 have been investigated, by measuring the plasma levels of diflunisal after oral administration of 0.04 mmol/kg p.o. in the rat, this dose being close to the therapeutical one.

As shown by table 3, the compound 5 is well absorbed in the gastroenteric tract, with a bioavailability with respect to diflunisal of about 79%.

It is differentiated from diflunisal by a greater latence time, by a definitely lower plasma peak and by a significatively longer halflife time.

TABLE 3

Pharmacokinetic parameters calculated on the basis of the plasma levels of diflunisal after administration of S-[2',4'-difluoro-4-hydroxy-(1,1'-diphenyl)-3-carbonyl]-N-acetyl-L-cysteien (compound 5) and of diflunisal at the orgal dose of 0.04 mmol/kg[1] in the rat.

| TREATMENT | C max | 'C max | $t_{\frac{1}{2}}$ | o→oo |
|---|---|---|---|---|
| | μg $ml^{-1}$ | h | h | μg.h.$ml^{-1}$ |
| Compound 5 | 15.0 | 8 | 5.8 | 271.96 |
| Diflunisal | 39.7 | 3 | 4.2 | 344.78 |

Also the tissue levels of diflunisal in the lungs have been measured since these tissues represent the sites of the therapeutical action foreseen for the compound 5.

The behaviour of these levels in the lungs is alike to that observed in the plasma, but in this case the AUCO→$24^h$ of compound 5 is higher than that of diflunisal and precisely is 105%. Such a value, if compared with that relating to the plots of the plasma levels indicates a preferential tropism of the compound 5 for the lung tissue.

The presence in the compounds of the invention, in an equilibrated ratio, of anti-inflammatory and mucolytic activity corresponding to those of the diflunisal and of the N-acetylcysteine respectively, does already represent an advantageeous characteristic since it permits to obtain, in the afore said pathologies, a desirable double therapeutical action, through the administration of only one substance, which is easier to carry out than the extemporary association of the two aforesaid active principles.

However the most important advantages of the compounds of the invention with respect to the extemporary association of the two active principles are represented by the scarce toxicity of the compound 5, which is surprisingly less than that of the diflunisal and of an equimolar mixture of diflunisal and N-acetylcysteine, by the favorable pharmacokinetic properties (longer apparent halflife, preferential tropism for the lung tissue, etc.) and moreover by the lack of local irritating effects, which permits the use of the compounds of the invention in form of pharmaceutical preparation suitable, besides for the oral administration, also for the aerosol, rectal and injecting administration, these routes being on the contrary probhibited for the diflunisal, as confirmed by experiments carried out by the Applicant.

For these reasons the compounds of the present invention may find useful indications of use in the acute and chronical diseases of the tracheo-bronchial tract and in the influenzal and para-influenzal forms in which the respiratory organs are involved. The compounds of the invention are prepared as described in the following examples.

Example 1

s-[2',4'-difluoro-4-(2,2,2-trichloroethoxy-carbonyloxy)-(1,1'-diphenyl)-3-carbonyl]-N-acetylcysteine (compound 4 with $R''=CH_2—CCl_3$).

A suspension of 5 g (20 mmoles) of 2',4'-difluoro-4-hydroxy-(1,1'-dephenyl)-3-carboxylic acid (compound 2) in 60 ml of acetone cooled to −3°C by means of a refrigerant mixture is added by portions with 4 g (40 mmoles) of triethylamine by maintaining under strong stirring. The resulting solution is treated, under stirring, over about 15 minutes with 8.5 g (4 mmoles) of 2,2,2-trichloroethyl-chlorocarbonate. The internal temperature increases from −3°C to 0°C and a white precipitate of triethylamine hydrochloride is gradually formed.

At the end of the addition the stirring is continued up to a total of 35 minutes of reaction, the internal temperature being maintained below 0°C.

A solution of 3.2 g (20 mmoles) of N-acetyl-L-cysteine and of 2 g (20 mmoles) of triethylamine in 15 ml of acetone is added, dropwise and under stirring over 15 minutes, to the acetonic solution of the mixed anhydride (3) cooled to 0°C. During the dropwise addition the internal temperature increases to 5°C. At the end, the internal temperature is left to increase up to 20°C and the mixture is still stirred for 4 hours. The suspension is filtered under vacuum to remove the precipitated triethylamine hydrochloride and the

acetonic filtrate is evaporated to dryness under vacuum to get an oily dense residue (9 g; yield: 78.9%). This residue is dissolved in 65 ml of chloroform and the solution is washed with a saturated aqueous solution of NaCl, dehydrated over $CaCl_2$ and evaporated under vacuum.

The residue is treated with di-isopropylether until the dissolution is almost completed.

After keeping at rest for a night an abundant, crystalline and colorless precipitate is separated, which is recovered by filtration.

The compound melts at 118—119°C (Kofler) and at the analyses (CSS, IR, CHN) is found to be the desired pure thioester.

Example 2

S-[2',4'-difluoro-4-(ethoxycarbonyloxy)-(1,1'-diphenyl)-3-carbonyl]-N-acetyl-L-cysteine (Compound 4 with $R''=C_2H_5$).

According to the conditions described in the example 1, 2.5 g (0,01 moles) of 2',4'-difluoro-4-hydroxy-(1,1'-diphenyl)-3-carboxylic acid (compound 2) in 30 ml of acetone are reacted with 2 g, i.e. 2.8 ml, (0.02 mol) of triethylamine and 2.17 g, corresponding to 1.9 ml, of ethylchlorocarbonate.

The acetone suspension of the thus obtained mixed anhydride is added under stirring with the solution of 1.6 g (0.01 mol) of N-acetyl-L-cysteine and 1 g (0.01 mol) of triethylamine in 15 ml acetone.

During the addition the internal temperature increases from −5°C to 2°C.

The temperature is left to raise up to 20°C and then is maintained under stirring for 8 hours. After filtration to separate the triethylamine hydrochloride precipitate, the filtrate is evaporated under vacuum. The resinous residue is dissolved in 70 ml water and the solution is made acidic under cool conditions and under vigorous stirring with 1N HCl.

The waxy precipitate upon remaining at rest under cool conditions takes crystalline aspect and is separated by filtration.

Yield: 4.2 g of raw product (89%).

The product is purified by hot dissolution in ether and precipitated again by concentration and resting at room temperature. M.p. 114°C (Kofler).

Example 3

S-[2',4'-difluoro-4-hydroxy-(1,1'-diphenyl)-3-carbonyl]-N-acetyl-L-cysteine (compound 5).

*Method A — hydrogenolysis with Zn in glacial acetic acid*

A solution of 2.78 g (4.8 mmoles) of S-[2',4'-difluoro-4-(2,2,2-trichloroethoxycarbonyloxy)-(1,1-diphenyl)-3-carbonyl]-N-acetyl-L-cysteine (compound 4 with $R''=CH_2CCl_3$) in 30 ml glacial acetic acid is added by portions, under vigorous stirring, with 2.5 g (38.2 gram atoms) of Zn as powder, freshly activated through washing with 10% CHI, under the external control of an ice-water bath.

At the end of the addition a stirring is carried out at room temperature (between 20° and 25°C) for 4 hours.

The mixture is filtered under vacuum and washed on the filter with glacial acetic acid.

The acetic filtrate is dropwise added under stirring to 80 ml of cool water whereby a colloidal colorless suspension is obtained.

After further stirring for 30 minutes under cool conditions, the mixture is filtered under vacuum, the residue is washed several times with water on the filter and, lastly, well squeezed under vacuum.

Then it is dried under vacuum in the presence of sodium lime until a constant weight is obtained. The resulting dry product is washed in a mortar with petroleum ether (b.p. 40—60°C), thereafter with ether and, lastly, with methylene chloride.

A further purification can be carried out by crystallization from chloroform and filtration of the hot solution, mainly for the removal of any trace of inorganic impurities. There are obtained 4.1 g of a crystalline colorless substance, melting at 104°—105°C (Kofler). Yield: 73%.

*Method B — Catalytic hydrogenolysis*

2.55 g of S-[2',4'-difluoro-4-(2',2',2'-trichloroethoxycarbonyloxy)-(1,1'-diphenyl)-3-carbonyl]-N-acetyl-L-cysteine (compound 4 with $R''=CH_2CCl_3$) in 42 ml of methanol in the presence of 3 drops of glacial acetic acid and of 0.25 g of Pd/C 10% are treated with hydrogen at room temperature and pressure: the absorption of hydrogen takes place in few minutes.

After filtration and evaporation of the filtrate, the residue seems to be formed by a mixture of several components, from which, through a purification method like that described for the Method A, 110 mg of the foreseen product are isolated having an acceptable purity degree.

Example 4

S-[2',4'-difluoro-4-hydroxy-(1,1'-diphenyl)-3-carbonyl]-N-acetyl-L-cysteine (compound 5)

Under the conditions described in the example 1, 214 g (0.855 mol) of 2',4'-difluoro-4-hydroxy-(1,1'-diphenyl)-3-carboxylic acid *(2)* in 2300 ml of acetone are reacted with 194.6 g equal to 268 ml (1.923 mol) of triethylamine and 407 g equal to 259 ml (1.923 mol) of 2,2,2-trichloroethylchlorocarbonate. At the end of the reaction, the reaction mixture is filtered under vacuum to separate the crystalline precipitate of triethylamine hydrochloride and the filtrate is evaporated under vacuum (1—2 mmHg) at room temperature.

The oily residue is taken with 300 ml of petroleum ether (b.p.: 40—60°C) and the suspension is evaporated again under vacuum.

The operation is repeated to remove any excess of chlorocarbonate still present. The oily residue, consisting of the mixed anhydride of formula 3 (with $R''=CH_2—CCl_3$) is dissolved in 300 ml of acetone and the resulting solution is added over 2 hours under stirring, so that the internal temperature increases from 5°C 14°C, to 139.5 g (0.855 mol) of N-acetyl-L-cysteine dissolved in 535 ml of acetone in the presence of 86.5 g equal to 119 ml (0.855 mol) of triethylamine under the condition described in the example 1.

At the end of the addition, the resulting mixture is stirred at 14—16°C for 3 hours.

After cooling at 5°C the reaction mixture is added with 30 ml of triethylamine, then maintained at rest at −10°C for 40 hours during which an abundant crystalline precipitate is separated, consisting of the triethylamine salt of S-[2′,4′-difluoro-4-hydroxy-(1,1′-diphenyl)-3-carbonyl]-N-acetyl-L-cysteine (compound 5′), which is isolated by filtration under vacuum. A further amount of this salt is recovered through concentration of the filtrate and further remaining at rest at −10°C.

155 of the triethylamine salt of the compound 5 (compound 5′) are isolated on the whole, it melting with decomposition at 165—168°C. This salt is added under vigorous stirring within about 10 minutes to 2400 ml of 1% HCl cooled to 0—1°C. The obtained crystalline precipitate is separated by filtration under vacuum, washed with water and lastly dried until achieving constant weight at 50°C under vacuum in the prsence of $P_2O_5$.

112 g (yield: 33%) of raw compound 5 are obtained, which are purified by crystallization from acetone (2100 ml) to isolate 87.2 g of S-[2′,4′-difluoro-4-hydroxy-(1,1′-diphenyl)-3-carbonyl]-N-acetyl-L-cysteine (compound 5) with a purity degree of ≥99% in form of a colorless crystalline solid, with a yield of 25,8% with respect to the starting diflunisal.

The compound melts at 180—182°C (Kofler) and the structure thereof is confirmed through the elemental analysis and the IR and NMR spectra (in dimethylsulfoxide). By treatment of the reaction liquors (acetonic filtrate resulting from the isolation of the triethylamine salt), through evaporation to dryness, alkaline hydrolysis of the residue with 10% alcoholic KOH, isolation by means of acidification with diluted HCl and purification, according to the standard techniques, 130 g of the starting diflunisal can be recovered.

The effective amount of diflunisal involved in the preparation is thus reduced to 84g (0.336 mol) and, with respect to such an amount, the final yield of pure compound 5 is 65.6%.

### Example 5

N-[2′,4′-difluoro-4-acetoxy-(1,1′-diphenyl)-3-carbonyl]-S-carboxymethylcysteine dimethyl ester (compound 7).

A solution of 2.44 g (0.001 mol) of S-carboxymethylcysteine dimethyl ester monohydrochloride, prepared according to the method disclosed in the literature (Mamalis et al, J. Chem. Soc. 1960, 2908), in 25 ml of chloroform is added, under stirring and cooling with an ice bath, with 1.01 g (0.01 mol) of triethylamine and, after 15 minutes, with 2.92 g (0.01 mol) of 2′,4′-difluoro-4-acetoxy-(1,1′-diphenyl)3-carboxylic acid (compound 6) and 2.06 g (0.01 mol) of dicyclohexylcarbodimide.

Stirring under cooling is still continued for 1 hour, during which a colorless precipitate of dicyclohexylurea is progressively formed, and then the reaction mixture is maintained at room temperature for 6 hours more. After filtration to remove the precipitate the chloroformic filtrate is poured off in a separating funnel and subsequently washed with portions of water (10 ml), 2.5% HCl (15 ml), 2.5% $NaHCO_3$ (15 ml) and lastly with water again (15 ml). After dehydration on Ca $Cl_2$ and evaporation under vacuum, the residue is taken with ether, wherein most of it is dissolved.

The undissolved part is separated by filtration and the ethanol filtrate is evaporated, the residue being then washed with acetone to give 4.15 g (yield: 86%) of a fluid resinous product.

It is an unitary substance (CSS) (see FO137) the IR spectrum of which corresponds to that of the sought products, which is subjected as such to the subsequent hydrolysis.

### Example 6

N-[2′,4′-difluoro-4-hydroxy-(1,1′-diphenyl)-carbonyl]-S-carboxymethylcysteine (conpound 8).

2 g (4.15 mmol) of the dimethylester obtained in the preceeding example 5 are taken with 15 ml of 2N NaOH and 5 ml of acetone and the resulting solution is maintained at rest for 3 days.

It is cooled with ice, made acidic with 5N HCl and lastly the suspension is extracted with ethyl acetate. The solid residue is washed several times with ether, until it takes a crystalline aspect.

Yield: 1.1 g corresponding to 64%; m.p. 97—99°C (Kofler)

### Example 7

S-[2′,4′difluoro-4-acetoxy-(1,1′-diphenyl)-carbonyl]-N-acetyl-L-cysteine (compound 10).

4.8 g of chloride of 2′,4′-difluoro-4-acetoxy-(1,1′-diphenyl)-3-carboxylic acid (compound 9) (15.5 mmol) dissolved into 9 ml of dimethlformamide (DMF) are dropwise added over 20 minutes under stirring to the solution of 2.53 g (15.5 mmol) of N-acetyl-L-cysteine and 3.95 g (387 mmol) of $N(C_2H_5)$ in 15 ml of DMF.

By means of an external bath of water-ice the internal temperature is controlled so that during the addition it is increased from 10°C to 25°C. The mixture is stirred for 3 hours at room temperature, then it is added under vigorous stirring and in small portions to 100 ml of cold water.

The resulting clear solution is made acidic with 5N HCl to get a resinous precipitate, which is decanted and taken again with 100 ml of cold water.

The resulting suspension is extracted with 100 ml of chloroform, and the organic extract is washed with water and dehydrated over $CaCl_2$.

After evaporation under vacuum, 6 g (yield: 88%) are obtained of a dense, straw yellow coloured resin, which extracted with boiling ether and then with boiling benzene.

The two extracts are maintained at rest at room temperature, thus being ocoled and concentrated, whereby several portions of a colorless crystalline precipitate are obtained.

m.p. 150—151°C (Kofler) (after further crystallization form ether).

The chemical identity and the purity of the compound have been controlled through chemical and laboratory analysis.

Example 8

S-[2',4'-difluoro-4-hydroxy-(1,1'-diphenyl)-3-carbonyl]-N-acetyl-L-cysteine lysine salt.

3.95 g of S-[2',4'-difluoro-4-hydroxy-(1,1'-diphenyl)-3-carbonyl]-N-acetyl-L-cysteine (5) are added with 2.92 g of lysine base as a 50% aqueous solution, 8 ml of water and 20 ml of acetone; the mixture is stirred until a clear solution is obtained and filtered.

Under stirring the mixture is diluted up to 100 ml with acetone, cooled to −10°C for a night, filtered and dried in a stove at 50—60°C under vacuum. A white, water soluble product is obtained having melting point of 179—184°C, with a yield of 92%.

The pharmaceutical compositions contemplated by the present invention include all the aforesaid forms and formulations which are prepared with the standard ingredients (exceipients, vehicles, etc.) and according to the standard pharmaceutical techniques.

As regards the therapeutical use of the compound of the invention, there are foreseen daily administrations of dosages of 500 to 1500 mg of active compound in 2—3 times, these dosages being valable for all the administration routes.

**Claims**

1. Compounds having the formula:

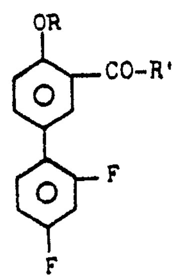

I

wherein

R=H, $—COCH_3—COOC_2H_5$, $—COOCH_2$, $—CCl_3$ and

R'=$—S—CH_2—CH(NHCOCH_3)—COOH$, $—NH—CH(CH_2—S—CH_2—COOH)—COOH$

and their non toxic and pharmaceutically acceptable salts with organic and inorganic bases.

2. S-[2',4'-difluoro-4-hydroxy-(1,1'-diphenyl)-3-carbonyl]-N-acetyl-L-cysteine according to claim 1.

3. N-[2',4'-difluoro-4-hydroxy-(1,1'-diphenyl)-3-carbonyl]-S-carboxymethylcysteine according to claim 1.

4. S-[2',4'-difluoro-4-(ethoxycarbonyloxy)-(1,1'-diphenyl)-3-carbonyl]-N-acetyl-L-cysteine according to claim 1.

5. S-[2',4'-difluoro-4-(2,2,2-trichloroethyoxy-carbonyloxy)-(1-1'-diphenyl)-3-carbonyl]N-acetyl-L-cysteine according to claim 1.

6. N-[2',4'-difluoro-4-acetoxy-(1,1'-diphenyl)-3-carbonyl]-S-carboxymethylcysteine dimethylester according to claim 1.

7. S-[2',4'-difluoro-4-acetoxy-(1,1'-diphenyl)-3-carbonyl]-N-acetyl-L-cysteine according to claim 1.

8. Lysine salt of S-[2',4'-difluoro-4-hydroxy-(1,1'-diphenyl)-3-carbonyl]-N-acetyl-L-cysteine according to claim 1.

9. A process for the preparation of the compounds according to claim 1, wherein

$$R' = S—CH_2—CH(—NH—CO—CH_3)—COOH$$

characterized in that 2′,4′-difluoro-4-hydroxy-1,1′-diphenyl)-3-carboxylic acid is converted to the corresponding mixed anhydride by treatment with 2,2,2-trichloroethyl-chlorocarbonate or ethylchlorocarbonate, the mixed anhydride being in turn converted, by reaction with N-acetylcysteine, to the O-alkylcarbonate thioester, from which the desired compound is obtained through hydrogenolysis.

10. A process according to claim 9, characterized in that said carboxylic acid is treated with 2,2,2-trichloroethyl-chlorocarbonate or ethylchlorocarbonate and the reaction for the preparation of the mixed anhydride is carried out in the presence of an acid acceptor.

11. A process according to claim 10, characterized in that said acid acceptor is triethylamine.

12. A process according to claim 9, characterized in that said reaction for the preparation of the mixed anhyride is carried out in the presence of an excess both of the alkylchlorocarbonate and of the triethylamine, the mixed anhydride is isolated and reacted with N-acetyl-L-cysteine, in the presence of triethylamine, whereafter, by a subsequently addition of an excess of triethylamine, the desired compound is isolated in form of triethylamine salt without the need of hydrogenolysis.

13. A process for the preparation of the compounds of claim 1, wherein

$$R'=—NH—CH(—CH_2—S—CH_2—COOH)—COOH$$

characterized in that 2′,4′-difluoro-4-acetoxy-(1,1′-diphenyl)-3-carboxylic acid is condensed with S-carboxymethylcysteine dimethylester in the presence of dicyclohexylcarbodiimide and the resulting product is subjected to alkaline hydrolysis to obtain the compound of claim 3.

14. A process for the preparation of the compounds of claim 1, wherein,

$$R=—COCH_3 \text{ and } R'=S—CH_2—CH(—NHCOCH_3)—COOH$$

characterized in that 2′,4′-difluoro-4-acetoxy-(1,1′-diphenyl)-3-carboxylic acid is converted to the corresponding acid chloride, the latter being then reacted with N-acetyl-L-cysteine to obtain the compound of claim 7.

15. Pharmaceutical composition, having particularly anti-inflammatory and mucolytic activity, chracterized by containing, as the active ingredient, a compound according to claim 1, together with conventional excipients, vehicles and the like.

16. Pharmaceutical composition according to claim 15, characterized by being in form suitable for oral administration.

17. Pharmaceutical composition according to claim 15, characterized by being in form suitable for aerosol administration.

18. Pharmaceutical composition according to claim 15, characterized by being in form of suppositories.

19. Pharmaceutical composition according to claim 15, characterized by being in injectable form.

**Patentansprüche**

1. Verbindungen mit der Formel:

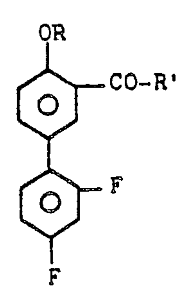

worin

$$R = H, —COCH_3—COOC_2H_5, —COOCH_2—CCl_3 \text{ und}$$

$$R' = -S-CH_2-\underset{\displaystyle NHCOCH_3}{\underset{|}{CH}}-COOH,\quad -NH-\underset{\displaystyle CH_2-S-CH_2-COOH}{\underset{|}{CH}}-COOH$$

und ihre nichtoxischen und pharmazeutisch verträglichen Salze mit organischen und anorganischen Basen.

2. S—(2',4'-Difluor-4-hydroxybiphenyl-3-ylcarbonyl)-N-acetyl-cystein nach Anspruch 1.

3. N—(2',4'-Difluor-4-hydroxybiphenyl-3-ylcarbonyl)-S-carboxymethylcystein nach Anspruch 1.

4. S—(2',4'-Difluor-4-(ethoxycarbonyloxy)-biphenyl-3-ylcarbonyl)-N-acetyl-cystein nach Anspruch 1.

5. S—(2',4'-Difluor-4-(2,2,2-trichlorethoxycarbonyloxy)-biphenyl-3-ylcarbonyl)-N-acetyl-cystein nach Anspruch 1.

6. N—(2',4'-Difluor-4-acetoxybiphenyl-3-ylcarbonyl)-S-carboxymethylcysteindimethylester nach Anspruch 1.

7. S—(2',4'-Difluor-4-acetoxybiphenyl-3-ylcarbonyl)-N-acetyl-cystein nach Anspruch 1.

8. Lysinsalz von S—(2',4'-Difluor-4-hydroxybiphenyl-3-ylcarbonyl)-N-acetyl-cystein nach Anspruch 1.

9. Verfahren zur Herstellung der Verbindungen nach Anspruch 1, worin

$$R' = -S-CH_2-\underset{\displaystyle NHCOCH_3}{\underset{|}{CH}}-COOH,$$

dadurch gekennzeichnet, daß 2',4'-Difluor-4-hyroxybiphenyl-3-carbonsäure durch Behandlung mit Chlorameisensäure-2,2,2-trichlorethylester oder Chlorameisensäureethylester in das entsprechende gemischte Anhydride umgewandelt wird, wobei das gemischte Anhydrid seinereits durch Umsetzung mit N-Acetylcystein in den O-Alkylcarbonat-thioester umgewandelt wird, aus dem durch Hydrogenolyse die gewünschte Verbindung erhalten wird.

10. Verfahren nach Anspruch 9, dadurch gekennzeichnet, daß die erwähnte Carbonsäure mit Chlorameisensäure-2,2,2-trichlorethylester oder Chlorameisensäreethylester behandelt und die Umsetzung für die Herstellung des gemischten Anhydrids in Gegenwart eines Säureakzeptors durchgeführt wird.

11. Verfahren nach Anspruch 10, dadurch gekennzeichnet, daß der erwähnte Säureakzeptor Triethylamin ist.

12. Verfahren nach Anspruch 9, dadurch gekennzeichnet, daß die erwähnte Umsetzung für die Herstellung des gemischten Anhydrids in Gegenwart eines Überschusses sowohl des Chlorameisensäurealkylesters als auch von Triethylamin durchgeführt wird und das gemischte Anhyrdrid isoliert und in Gegenwart von Triethylamin mit N-Acetyl-L-cystein umgesetzt wird, worauf durch eine nachfolgende Zugabe eines Überschusses von Triethylamin die gewünschte Verbindung in Form des Triethylaminsalzes isoliert wird, ohne daß eine Hydrogenolyse erforderlich ist.

13. Verfahren zur Herstellung der Verbindungen nach Anspruch 1, worin

$$R' = -NH-\underset{\displaystyle CH_2-S-CH_2-COOH,}{\underset{|}{CH}}-COOH$$

dadurch gekennzeichnet, daß 2',4'-Difluor-4-acetoxybiphenyl-3-carbonsäure in Gegenwart von Dicyclohexylcarbodiimid mit S-Carboxymethylcysteindimethylester kondensiert und das erhalte ne Produkt einer alkalischen Hydrolyse unterzogen wird, um die Verbindung nach Anspruch 3 zu erhalten.

14. Verfahren zur Herstellung der Verbindungen nach Anspruch 1, worin

$$R = -COCH_3 \text{ und } R' = -S-CH_2-\underset{\displaystyle NHCOCH_3}{\underset{|}{CH}}-COOH,$$

dadurch gekennzeichnet, daß 2',4'-Difluor-4-acetoxybiphenyl-3-carbonsäure in das entsprechende Säurechlorid umgewandelt wird, wobei dieses dann mit N-Acetyl-L-cystein umgesetzt wird, um die Verbindung nach Anspruch 7 zu erhalten.

15. Arzneimittel, das insbesondere entzündungschemmende und schleimlösende Wirkung zeight, dadurch gekennzeichnet, daß es als Wirkstoff eine Verbindung nach Anspruch 1 zusammen mit überlichen Bindemitteln, Lösungsmitteln bzw. Trägern u.dgl. enthält.

16. Arzneimittel nach Anspruch 15, dadurch gekennzeichnet, daß es in für orale Verabreichung geeigneter Form vorliegt.

17. Arzneimittel nach Anspruch 15, dadurch gekennzeichnet, daß es in für eine Verabreichung als Aerosol geeigneter Form vorliegt.

18. Arzneimittel nach Anspruch 15, dadurch gekennzeichnet, daß es in Form von Zäpfchen vorliegt.

19. Arzneimittel nach Anspruch 15, dadurch gekennzeichnet, daß es in injizierbarer Form vorliegt.

**Revendications**

1. Composés répondant à la formule:

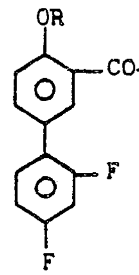

R = H, —$COCH_3$—$COOC_2H_5$, —$COOCH_2$, —$CCl_3$ et
R' = —S—$CH_2$—CH—COOH (CH substitué par $NHCOCH_3$), = —NH—CH—COOH (CH substitué par $CH_2$—S—$CH_2$—COOH)

ainsi que leurs sels non toxiques et acceptables en pharmacie, avec des bases organiques et inorganiques.

2. S-[2',4'-difluoro-4-hydroxy-(1,1'-diphényl)-3-carbonyl]-N-acétyl-L-cystéine suivant la revendication 1.

3. N-[2',4'-difluoro-4-hydroxy-(1,1'-diphényl)-carbonyl]-S-acarboxyméthylcystéine suivant la revendication 1.

4. S-[2',4'-difluoro-4-éthoxycarbonyloxy-(1,1'-diphényl)-3-carbonyl]-N-aceztyl-L-cystéine suivant la revendication 1.

5. S-[2',4'-difluoro-4-(2,2,2-trichloroéthyoxycarbonyloxy)-(1,1'-diphényl)-3-carbonyl]-N-acétyl-L-cystéine suivant la revendication 1.

6. N-[2',4'-difluoro-4-acétoxy-(1,1'-diphényl)-3-carbonyl]-S-carboxyméthylcystéine, ester diméthylique suivant la revendication 1.

7. S-[2',4'-difluoro-4-acétoxy-(1,1'-diphényl)-3-carbonyl]-N-acétyl-L-cystéine suivant la revendication 1.

8. Sel de lysine de S-[2',4'-difluoro-4-hydroxy-(1,1'-diphényl)-3-carbonyl]-N-acétyl-L-cystéine suivant la revendication 1.

9. Un procédé our la preparation des composés suivant la revendication 1, ou:

R' = S—$CH_2$—CH—COOH (CH substitué par NH—$COCH_3$)

charactérisé en ce que l'acide 2',4'-difluoro-4-hydroxy-1,1'-diphényl)-3-carboxylique est transformé en l'anhydride mixte correspondant par traitement avec du chlorocarbonate de 2,2,2-trichloroéthylechlorocarbonate d'éthyle, l'anhydride mixte étant à son tour converti, par réaction avec N-acétylcystéine, en thioester O-alkylcarbonate duquel on obtient le composé désiré par hydrogénolyse.

10. Un procédé suivant la revendication 9, caractérisé en ce que le did ou du acid carboxylique est traité avec chlorocarbonate de 2,2,2- trichloroéthyle ou chlorocarbonate d'éthyle et la réaction pour la préparation de l'anhydride mixte est réalisée en présence d'un accepteur d'acide.

11. Un procédé suivant la revendication 10, caractérisé en ce que le did accepteur d'acide est triéthylamine.

12. Un procede suivant la revendication 9, caractérisé en ce que la dite reaction pour la préparation de l'anhyride mixte est réalisée en présence d'un excés de chlorocarbonate d'alkyle et de triéthylamine, on isole l'anhydride mixte et on fait réagir avec de la N-acétyl-L-cystéine en présence de triéthylamine, aprés quois, avec une nouvelle addition d'un excés de triéthylamine, on isole le composé désiré sous forme du sel de triéthylamine sans nécessité d'une hydrogénolyse.

13. Un procédé de préparation des composés suivant la revendication 1, ou:

R' = —NH—CH—COOH (CH substitué par $CH_2$—S—$CH_2$—COOH)

caractérisé en ce qu'on condense l'acide 2',4'-difluoro-4-acétoxy-(1,1'-diphényl)-3-carboxylique avec ester diméthylique de S-carboxyméthylcystéine en présence de dicyclohexylcarbodiimide et le produit résultant est soumis à hydrolyse alcaline pour obtenir le composé de la revendication 3.

14. Un procédé de préparatioin des composés suivant la revendication 1, ou:

$$R = -COCH_3 \quad R' = -S-CH_2-\underset{\substack{|\\ NHCOCH_3}}{CH}-COOH$$

caractérisé en ce que l'on transforme l'acide 2'4'-difluoro-4-acétoxy-(1,1'-diphényl)-3-carboxylique en le chlorure d'acide correspondant et ensuite on fait reagir ce dernier avec N-acétyl-L-cystéine pour obtenir le composé de la revendication 7.

15. Composition pharmaceutique ayant aen particulier une activité antiinflammatoire et une activité mucolytique, caractérisé en ce qu'elle contienne, comme ingrédient actif, un composé suivant la revendication 1, avec courants excipients, vehicles et autres.

16. Composition pharmaceutique suivant la revendication 15, caractérisée en ce qu'elle est sous une forme permettant une administration par voie orale.

17. Composition pharmaceutique suivant la revendication 15, caractérisée en ce qu'elle est sous une forme permettant une administration par voie d'aérosol.

18. Composition pharmaceutique suivant la revendication 15, caractérisée en ce qu'elle est sous la forme de suppositoires.

19. Composition pharmaceutique suivant la revendication 15, caractérisée en ce qu'elle est sous la forme injectable.